# EUROPEAN PATENT SPECIFICATION

(11) **EP 4 156 972 B1**
(45) Date of publication and mention of the grant of the patent: **12.11.2025**
(21) Application number: 21727145.1
(22) Date of filing: 20.05.2021
(51) Int. Cl.: A23L 33/00, A21D 13/04, A21D 13/045, A21D 13/047, A21D 13/41, A23L 33/20, G06Q 30/00, G06Q 30/02, G09B 19/00, G16H 20/60

(54) **METHOD FOR DETERMINING OPTIMIZED COMPOSITIONS OF FOOD PRODUCTS**
VERFAHREN ZUR BESTIMMUNG OPTIMIERTER ZUSAMMENSETZUNGEN VON NAHRUNGSMITTELN
MÉTHODE POUR DÉTERMINER DES COMPOSITIONS OPTIMISÉES DE PRODUITS ALIMENTAIRES

(30) Priority: 29.05.2020 EP 20177558
(43) Date of publication of application: 05.04.2023
(73) Proprietor: Nutriomix GmbH, 88400 Biberach an der Riss (DE)
(72) Inventor: CAYLI, Aziz, 10629 Berlin (DE)
(74) Representative: Wohlfahrt, Jan Günther
(86) International application number: PCT/EP2021/063454
(87) International publication number: WO 2021/239571

(56) References cited:
- CA-A1- 3 005 341
- DE-U1- 202010 012 766
- US-A1- 2006 085 272
- US-A1- 2016 103 834
- US-A1- 2018 233 223
- US-A1- 2018 279 628
- US-A1- 2019 008 191
- DATABASE GNPD [online] MINTEL; 22 April 2020 (2020-04-22), ANONYMOUS: "Original Cauliflower Pizza Crust", XP055738014, retrieved from www.gnpd.com Database accession no. 7563645
- DATABASE GNPD [online] MINTEL; 20 March 2020 (2020-03-20), ANONYMOUS: "Pizza Crust Made with Superfood Ingredients", XP055738016, retrieved from www.gnpd.com Database accession no. 7454461
- ANONYMOUS: "Five Vegetable Pizza", GNPD, 1 December 2007 (2007-12-01), XP002638820

## Description

Industry illnesses, such as cardiovascular disease, high blood pressure, diabetes mellitus type 2 (DMT2), some cancers and poor bone health have been shown to have a certain degree of correlation with eating patterns. The proportion of overweight or obese people is alarmingly increasing. Poor eating patterns correspond to excessive consumption of certain nutrients, like saturated fat or sugar, but also to low intake of other important nutrients, like vitamin B6 or potassium.

The potential impact of diet to the health status is enormous when combined with not smoking and regular physical activity. Previous studies have shown that about 82 percent of heart attacks, about 70 percent of strokes, over 90 percent of type 2 diabetes, and over 70 percent of colon cancer, could be prevented with the right dietary choices.

A large variety of diets is available for different purposes, e.g. to lose weight, to gain weight, to build muscle, to treat diabetes, etc. Even though some diets promote expected effects in short term, in longer term almost all of them fail meeting the goals. Therefore, the number of people with nutrition related illnesses increases worldwide. Apparently, the problems associated with poor eating patterns could not be solved. Contrarily, diet related problems are increasing.

One reason for failing diets might relate to the reductionist thinking. In order to understand the problem, scientists often tend to isolate one aspect of the problem and evaluate it isolated from the rest of variables. The reductionist thinking has dominated up to now the diets and the diet related food products. Diets focusing on reduction of carbohydrates, on reduction of fats or on increase of the protein content in food products are typical examples of reductionist thinking. However, it has to be considered that the human body is complex and foods are complex as well. Thus, two complex systems start interacting with each other when food is consumed. Many parameters start to influence each other and to act synergistically. It is impossible to isolate one parameter and try to solve the problem only in light of that single aspect.

Reductionist thinking is illustrated in following example: There are 11 different saturated fatty acids, 6 mono unsaturated fatty acids, 6 poly unsaturated n-6 fatty acids and 5 poly unsaturated n-3 fatty acids published in foods. These fatty acids are the ones which were measurable with the analytical tools today. Certainly, there is a larger number of unknown fatty acids in foods which are not detectable with current analytical tools. Fatty acids of foods are complex and the exact functions of most of the fatty acids are still unknown. Despite this, the scientific world tends to reduce the discussion to omega-3 fatty acids (good fat) and to saturated fatty acids (bad fats). Even though a food consists of thousands of nutrients each with a distinct function, the food is reduced to its omega-3 fatty acid content only. As omega-3 fatty acids were shown to have a positive effect on health, it is recommended to consume foods with omega-3 fatty acids or omega-3 fatty acid pills. In contrast, foods comprising saturated fatty acids are recommended to be avoided.

The problem might be solved partly by holistic approaches. Holistic approaches consider consuming whole foods instead of isolated food parts. There are efforts to consider macronutrients in a diet and to avoid imbalance of macronutrients in foods (US 2019/0159505 A1). Even that approach is only partly holistic as focus is particularly set on macronutrients such as carbohydrates, fats, and proteins. However, these macronutrients merely represent a fraction of the complex cellular requirements. Cells need hundreds or thousands of different nutrients to function optimally. Required salts, trace elements, vitamins all together are not considered in current approaches. Even focusing on a particular macronutrient, e.g. "protein" cannot bring expected positive results. It has to be considered that in case there is an imbalance in the amino acid composition of food due to its protein composition, that food cannot be nutritionally optimal for the body even if the protein concentration itself is optimal. It is therefore necessary to go one further step in detail and to balance at least the amino acid composition, fatty acid composition, vitamin composition and mineral composition in food products.

Different attempts are published to optimize the nutritional profile of foods. Most of these optimization studies focus to caloric intake from carbohydrates, proteins or fats. Current food products are often supplemented with single isolated nutrients, e.g. vitamins, minerals or food fragments like whey or milk protein (WO 01/67889 A1). However, adding single nutrients into a food is a reductionist approach. Balancing the nutritional profile of foods is far more than balancing the caloric intake from macronutrients. Single nutrients are interacting with each other and generate positive or negative synergistic effects in the cells. For example, it has been demonstrated that proteins in combination with vitamin D and calcium together promote muscle formation. In first instance these single nutrients do not belong to the same category of nutrients. Proteins belong to macronutrients and are source of essential and non-essential amino acids. Thus, proteins are source of energy and contribute to regeneration. Vitamin D promotes vitality. Calcium is a mineral that is known to promote bone strength. However, only in combination those three single nutrients generate a synergy with totally different biological functions and promote muscle formation (US 10,455,853 B2). Thus, for optimal body function essential, semi-essential and non-essential nutrients need to be optimized in a similar way because nutrients work in a synergistic way.

Making healthier food choices can help prevent some illnesses. Food-based dietary guidelines, like the Dietary Guidelines for Americans, provide general population recommendations for healthy eating. These tend to be based on nutrient adequacy and reduced risk of diet-related diseases. In this way, dietary guidelines are published for example by Institute of Medicine (IOM) the Dietary Reference Index (DRI) or the Healthy Eating Index (HEI 2015). Such guidelines can drive nutrition education or menu planning. Recommendations for healthier food choices are divided in two levels: at the level of food categories, e.g., eat more fruits, eat less meat, etc. and at the level of nutrients, e.g. eat more omega-3 fatty acids, eat less saturated fatty acids, etc. However, customers have difficulty logistically to organize the food items from different food categories all the time or customer have difficulty to know whether/when nutritional recommended goals have been achieved. These generalized guidelines are difficult to praxis as well since customer need to know the nutrient profile of each food before consumption.

In order to help customers a number of software is available to balance their diets. The software usually recommends to the client certain foods to eat in calculated quantity. Customer record what has been consumed and the program calculates the nutritional balance and suggests the next meal for that specific person (US 2010/0198605 A1). Thus, customer will be monitored over time (WO 2016/050958 A1). Such approach has various drawbacks. First, the approach is customer specific and missing a general applicability. Second, the software suggests to the customer actually a behavioral change in eating habits. Behavior of a person can change in short term and customer might get positive results, but in long term, customer will fall back to its old behavioral patterns, such that the concept is not sustainable. Third, these approaches generate constant logistic stress to search for the specific food suggested by the software. In busy everyday life it is not simple to have the suggested food available at the required moment. Consequently, a person might often have the tendency to eat whatever is available at home and loose the motivation to exactly follow the recommendations. Fourth, people's freedom is restricted to freely choose a food. A person is required choose one of a few alternatives suggested by the software. In this regard, US 2010/0198605 A1 discloses a food selection system.

US 2006/085272 A1 discloses a system and method for helping users determine, evaluate, and/or place orders for nutritional blend formulations to be manufactured from available nutritional formulation materials.

US 2016/103834 A1 describes a scoring and ranking system for food recipes based on one or more culinary objectives.

Some publications represent some nutrients as healthy and the other nutrients as unhealthy. They recommend to reduce or even omit so called "unhealthy" nutrients, e.g. carbohydrates and increase "healthy" nutrients, e.g. proteins in foods. However, the healthiness of a nutrient is a function of its concentration, not of its presence or absence in food. A good example is the current high protein hype. Food industry produces products with higher and higher protein content because of the trend with little scientific evidence. There are up to 70% protein containing food products available in market. Costumers who eat more protein containing food products hope to gain muscles or lose weight. Even though they might achieve that goal in short term and they might get new health problems with their kidneys by detoxification of highly released ammonia or gout through high uric acid concentration in blood or increased body fat produced from excessive protein consumption. Another common approach of food industry to nutritionally balance the foods is to take any standard food and to supplement it with limiting nutrients. For example, muesli is supplemented with chemically synthesized vitamins. In this way, muesli seems to be nutritionally more balanced but this approach has following deficiencies. First, addition of a single nutrient or group of nutrients, e.g. vitamins can have either no or detrimental effect to cell metabolism. The basic idea was to balance the nutritional profile of a food product by supplementing it with an isolated nutrient. In reality, that food might not have an expected positive effect; even worse in some cases the supplementation might cause negative effect to the body. For example, it became a hype to supplement food with antioxidants because of many positive reports on their influence on health. However, a recent study demonstrated that supplementing the foods with strong antioxidants, vitamin E and N-acetyl cysteine increased tumor progression and reduced the survival in mouse (Sayin, et.al., Science Translational Medicine, 2014: Vol. 6, Issue 221, pp. 221). Secondly, a food is not nutritionally balanced if couple of single nutrients are added to the food.

All macronutrients and micronutrients need to be perfectly balanced in a perfect ratio to each other. All nutrients in a food work in a synergistic way to exhibit their positive effect. If only one of hundreds of nutrients is imbalanced, the expected positive effect might not occur. Currently, around 30 macronutrients and micronutrients are measured in foods and only a few macronutrients are balanced by food industry. On the other hand, any food consists of more than 30 single nutrients. A food can rather consists of many thousands of single nutrients. Therefore, supplementing a food by one or two single nutrients (e.g. vitamin E or N-acetyl cysteine or proteins) does not solve the balancing problem. In contrast, it might introduce a new, more dramatic imbalance because of its high dosage.

Accordingly, there is no publication about a food product which is balanced in broad context regarding macronutrients and micronutrients using natural raw materials. Published food products are designed considering only a few macronutrients or micronutrients. Published foods are supplied randomly with a few nutrients without taking care of balancing other nutrients or synergies. Published food products for improving health are mostly supplied with chemically synthetized single nutrients, not with naturally occurring nutrients, natural occurring foods or naturally occurring food raw materials.

Thus, there is a need to offer food products to clients, which do not necessitate behavioral changes, which customers can regularly buy in a simple way and which are healthier alternatives to the common food products in market. There is further a need to offer full freedom to clients to choose their preferred food products.

The present invention solves the technical problem by the subject-matter of the independent claims, in particular by providing a method for preparing a food product according to the present invention. The present invention is defined by the claims. Any subject-matter falling outside the scope of the claims is provided for information purposes only.

The present invention pertains to a computer-implemented method for preparing a food product, comprising the steps:
a) determining the composition of a food product, comprising the steps:
   i.) providing at least one database comprising nutrient profiles of raw materials from a natural source, a target food product and a target nutrient profile of the target food product,
      wherein the target nutrient profile is a specific group of at least target 6 nutrients each in a target concentration, wherein the target nutrient profile has a more balanced ratio of nutrients and a higher diversity of nutrients than a common food product corresponding to the target food product,
   ii.) applying an algorithm for determining the type and quantity of at least four different raw materials selected from the group consisting of grains, legumes, seeds, nuts, oils, fruits, vegetables, sea food, meat, meat products, milk and milk products in the target food product on a group of nutrient profiles of raw materials in the at least one database,
      wherein the algorithm has been trained using a machine learning procedure,
   iii.) providing the determined type and quantity of the at least four different raw materials of the target food product having a nutrient profile in which the nutrient concentrations of at least 6 nutrients differ from the corresponding nutrient concentrations in the target nutrient profile by at most 30%,
      wherein the types and quantities of the at least four different raw materials for preparing the target food product are provided in the form of a recipe,
b) preparing a food product having the composition determined in step a) from the at least four different raw materials.

Accordingly, the method for preparing a food product according to the present invention includes a computer-implemented method for determining the composition of a food product, in which at least one database comprising nutrient profiles of raw materials from a natural source, a target food product and a target nutrient profile of the target food product are provided in step i.), wherein the target nutrient profile is a specific group of at least 6 target nutrients each in a target concentration, and wherein the target nutrient profile has a more balanced ratio of nutrients and a higher diversity of nutrients than a common food product corresponding to the target food product. According to the present invention, a target food product and a target nutrient profile of the target food product can be freely chosen according to the specific requirements or individual preferences. Subsequently, an algorithm for determining the type and quantity of at least four different raw materials selected from the group consisting of grains, legumes, seeds, nuts, oils, fruits, vegetables, sea food, meat, meat products, milk and milk products in the food product is applied on a group of nutrient profiles of raw materials in the at least one database by using a technical device, in particular a computer. In this step, the algorithm determines the type of raw materials and the respective quantity of raw materials in a group of raw materials in the at least one database, which are required to obtain the target food product comprising at least four different raw materials and having a total nutrient profile which corresponds to the target nutrient profile, in particular in which the nutrient concentrations of at least 6 nutrients differ from the corresponding nutrient concentrations in the target nutrient profile by at most 30%. The determined type and quantity of raw materials of the food product having the nutritional profile is provided in the form of a recipe and can then be used to prepare a target food product having a nutrient profile corresponding to the target nutrient profile, in particular in which the nutrient concentrations of at least 6 nutrients differ from the corresponding nutrient concentrations in the target nutrient profile by at most 30%.

In a particularly preferred embodiment of the present invention, the at least one database comprising nutrient profiles of raw materials from a natural source, is a digital database. Preferably, the at least one database comprising nutrient profiles of raw materials from a natural source is a digital online database. In another preferred embodiment of the present invention, the at least one database comprising nutrient profiles of raw materials from a natural source is a digital offline database. Preferably, the at least one database is a SQL database.

Preferably, the at least one database comprising nutrient profiles of raw materials from a natural source is locally stored, in particular stored on a computer-readable storage medium. Preferably, the at least one database comprising nutrient profiles of raw materials from a natural source is locally stored, in particular stored on the computer-readable storage medium of a computer, tablet, smartphone, smartwatch or fitness tracking device. In another preferred embodiment of the present invention, the at least one database comprising nutrient profiles of raw materials from a natural source, is globally stored, in particular stored on a server.

Preferably, the at least one database comprising nutrient profiles of raw materials from a natural source, in particular the at least one digital database comprising nutrient profiles of raw materials from a natural source, is selected from the group consisting of U.S. department of agriculture database, European Food Information Resource database and The Swiss Food Composition Database.

Preferably, in step i.) at least two, preferably at least three, preferably at least four, preferably at least five, databases comprising nutrient profiles of raw materials from a natural source, in particular digital databases comprising nutrient profiles of raw materials from a natural source, are provided. In another preferred embodiment of the present invention, a database comprising nutrient profiles of raw materials from a natural source, in particular a single digital database comprising nutrient profiles of raw materials from a natural source is provided in step i.).

According to the present invention, the food product comprises at least four raw materials, preferably at least five raw materials, preferably at least six raw materials.

In a further preferred embodiment of the present invention, the nutrient profiles of the raw materials from a natural source in the at least one database comprise data on the type and quantity of nutrients, in particular micro- and macronutrients, in the raw materials.

In a particularly preferred embodiment of the present invention, the group of nutrient profiles of raw materials from a natural source encompasses nutrient profiles of all raw materials in the at least one database. Preferably, the group of nutrient profiles of raw materials from a natural source encompasses nutrient profiles of selected raw materials in the at least one database, preferably of raw materials selected from non-allergic raw materials, meat-free raw materials, raw materials free of animal products, kosher raw materials and/or halal raw materials. Thus, the method according to the present invention allows the selection of a group of certain specific raw materials from a natural source, which for example meet cultural or religious dietary practices, ethical dietary practices, medical dietary practices, allergenic dietary practices, and/or specific taste preferences.

In another preferred embodiment of the present invention, the target nutrient profile of the target food product provided in step i.) is improved in comparison to the nutrient profile of a common food product corresponding to the target food product. According to the present invention, the target nutrient profile of the target food product provided in step i.) has a more balanced ratio of nutrients and a higher diversity of nutrients than common food product corresponding to the target food product. Preferably, the target nutrient profile of the target food product provided in step i.) has a higher content of nutrients than common food product corresponding to the target food product. According to the present invention, the target nutrient profile of the target food product provided in step i.) has a more balanced ratio of nutrients than common food product corresponding to the target food product. Moreover, the target nutrient profile of the target food product provided in step i.) has a higher diversity of nutrients than common food product corresponding to the target food product. Preferably, the target nutrient profile of the food product provided in step i.) has lower calories than common food products corresponding to the target food product. Most preferably, the target nutrient profile of the food product provided in step i.) has higher diversity of nutrients and less calories than common food product corresponding to the target food product.

In a further preferred embodiment of the present invention, the target nutrient profile of the target food product provided in step i.) corresponds to the nutrient profile of a common food product corresponding to the target food product, wherein the target food product varies in at least one, preferably at least two, preferably at least three, preferably at least four, preferably at least five, raw material from the common food product.

Preferably, the target nutrient profile of the target food product provided in step i.) corresponds to the nutrient profile of a common food product corresponding to the target food product, wherein the quantity of the nutrients in the target food product is altered, in particular increased, in comparison to the common food product.

In another preferred embodiment, the target nutrient profile of the target food product provided in step i.) corresponds to the nutrient profile of a common food product corresponding to the target food product, wherein the concentration of the nutrients in the target food product is altered, in particular increased, in comparison to the common food product.

In a particularly preferred embodiment of the present invention, the target nutrient profile of the target food product provided in step i.) corresponds to the nutrient profile of a common food product corresponding to the target food product, wherein the quantity and the concentration of the nutrients in the target food product is altered, in particular increased, in comparison to the common food product.

In a preferred embodiment of the present invention, the target nutrient profile of the target food product provided in step i.) is a nutrient profile for a specific group of individuals or for a specific application. Preferably, the target nutrient profile of the food product provided in step i.) is a nutrient profile for a specific group of individuals. In a further preferred embodiment of the present invention, the target nutrient profile of the food product provided in step i.) is a nutrient profile for a specific application.

Preferably, the specific group of individuals is selected from the group consisting of infants, children up to the age of 12 years, adults up to the age of 50 years and adults older than 50 years. Particularly preferred, the specific group of individuals are infants, in particular infants up to the age of 2. In a further preferred embodiment of the present invention, the specific group of individuals are children, in particular children up to the age of 12. Preferably, the specific group of individuals are adults, in particular adults up to the age of 50. Preferably, the specific group of individuals are adults older than 50, preferably adults older than 60, preferably adults older than 70, preferably adults older than 80. In another preferred embodiment of the present invention the specific group of individuals are pregnant women. Preferably, the specific group of individuals are lactating women. In a particularly preferred embodiment of the present invention, the specific group of individuals are overweight individuals, in particular obese individuals. Preferably, the specific group of individuals are individuals who would like to gain muscle mass. In another preferred embodiment of the present invention, the specific group of individuals are individuals suffering from diabetes, cardiovascular diseases, cancer and/or Alzheimer's disease. Preferably, the group of specific individuals are individuals with an increased risk of developing diabetes, cardiovascular diseases, cancer and/or Alzheimer's disease. Accordingly, the method according to the present invention allows to determine the composition of a food product suitable to meet the nutrient requirements of different groups of individuals, in particular depending on for example the stage of life, genetic background, health condition and/or medication. In a particularly preferred embodiment of the present invention, the target nutrient profile of the food product provided in step i.) is a nutrient profile for a specific individual.

In a particularly preferred embodiment of the present invention, the specific group of individuals is a group of animals, in particular domestic animals, such as but not limited to pets or farm animals. Thus, the method according to the present invention allows to determine the composition of a food product suitable to meet the nutrient requirements of animals as well. For example, the composition of a food product determined by the method according to the present invention, can be the composition of a food product suitable to meet the nutrient requirements of cats, dogs, horses, cows, pigs, goats and/or sheep.

In another preferred embodiment, the target nutrient profile of the food product provided in step i.) is a gender-specific nutrient profile.

In a preferred embodiment of the present invention, the target nutrient profile encompasses nutrient values of at least 7 nutrients, preferably at least 8 nutrients, preferably at least 9 nutrients, preferably at least 10 nutrients, preferably at least 11 nutrients, preferably at least 12 nutrients, preferably at least 13 nutrients, preferably at least 14 nutrients, preferably at least 15 nutrients, preferably at least 16 nutrients, preferably at least 17 nutrients, preferably at least 18 nutrients, preferably at least 19 nutrients, preferably at least 20 nutrients, preferably at least 25 nutrients, preferably at least 30 nutrients, preferably at least 35 nutrients, preferably at least 40 nutrients, preferably at least 45 nutrients, preferably at least 50 nutrients, preferably at least 60 nutrients, preferably at least 70 nutrients.

Preferably, the target nutrient profile of the target food product is a nutrient profile providing at least 20%, preferably at least 25%, preferably at least 30%, preferably at least 35%, preferably at least 40%, preferably at least 45%, preferably at least 50%, preferably at least 55%, preferably at least 60%, preferably at least 65%, preferably at least 70%, preferably at least 75%, preferably at least 80%, preferably at least 85%, preferably at least 90%, preferably at least 95%, preferably 100%, of nutrients to meet the dietary reference intakes (DRI) or the recommended dietary allowances (RDA), in particular to meet the dietary reference intakes (DRI) or the recommended dietary allowances (RDA) of an individual, in particular of a specific group of individuals.

In a further preferred embodiment of the present invention, the target nutrient profile of the target food product is a nutrient profile providing 20%, preferably 30%, preferably 40%, preferably 50%, preferably 60%, preferably 70%, preferably 80%, preferably 90%, preferably 100%, of nutrients to meet the dietary reference intakes (DRI) or the recommended dietary allowances (RDA), in particular to meet the dietary reference intakes (DRI) or the recommended dietary allowances (RDA) of an individual, in particular of a specific group of individuals.

According to the present invention, the raw material is a material from a natural source. Preferably, the raw material is a naturally occurring raw material or a part of naturally occurring raw material. In a further preferred embodiment of the present invention, the raw material has been prepared in an industrial process, in particular is a processed raw material.

In a particularly preferred embodiment of the present invention, the target food product has a nutrient profile in which the nutrient values of at least 6 nutrients, preferably at least 7 nutrients, preferably at least 8 nutrients, preferably at least 9 nutrients, preferably at least 10 nutrients, preferably at least 11 nutrients, preferably at least 12 nutrients, preferably at least 13 nutrients, preferably at least 15 nutrients, preferably at least 16 nutrients, preferably at least 17 nutrients, preferably at least 18 nutrients, preferably at least 19 nutrients, preferably at least 20 nutrients, preferably at least 25 nutrients, preferably at least 30 nutrients, preferably at least 35 nutrients, preferably at least 40 nutrients, preferably at least 45 nutrients, preferably at least 50 nutrients, preferably at least 60 nutrients, preferably at least 70 nutrients, differ from the corresponding nutrient values in the target nutrient profile provided in step i.) by at most 50%, preferably at most 45%, preferably at most 40%, preferably at most 35%, preferably at most 30%, preferably at most 25%, preferably at most 20%, preferably at most 15%, preferably at most 10%, preferably at most 5%.

In another preferred embodiment of the present invention, the target food product has a nutrient profile in which the nutrient values of at most 10 nutrients, preferably at most 9 nutrients, preferably at most 8 nutrients, preferably at most 7 nutrients, preferably at most 6 nutrients, preferably at most 5 nutrients, preferably at most 4 nutrients, preferably at most 3 nutrients, preferably at most 2 nutrients, preferably at most 1 nutrient, differ from the corresponding nutrient values in the target nutrient profile provided in step i.) by at least 5%, preferably at least 10%, preferably at least 15%, preferably at least 20%, preferably at least 25%, preferably at least 30%, preferably at least 35%, preferably at least 40%, preferably at least 45%, preferably at least 50%.

Particularly preferred, the target food product has a nutrient profile in which the nutrient values of at most 5 nutrients, preferably at most 4 nutrients, preferably at most 3 nutrients, preferably at most 2 nutrients, preferably at most 1 nutrient, exceed the tolerable upper intake level (UL). Preferably, the nutrient value of no nutrient in the nutrient profile of the target food product exceeds the tolerable upper intake level (UL).

In a preferred embodiment of the present invention, the average deviation of the nutrient values in the nutrient profile of the target food product from the nutrient values in the target nutrient profile is at most 5%, preferably at most 10%, preferably at most 15%, preferably at most 20%, preferably at most 25%, preferably at most 30%, preferably at most 35%, preferably at most 40%.

According to the present invention, the algorithm for determining the type and quantity of different raw materials in the target food product has been trained with using a machine learning procedure.

Preferably, the machine learning procedure is based on an algorithm selected from the group consisting of linear regression, logistic regression, support vector machine, decision tree, random forest, K-nearest neighbors (kNN), K-means clustering, naive Bayes, principal component analysis (PCA), supersparse linear integer model (SLIM), neural network, gradient boosted tree regression.

According to the present invention, the types and quantities of raw materials for preparing the target food product having a nutrient profile in which the nutrient values of at least 6 nutrients differ from the corresponding nutrient values in the target nutrient profile by at most 30% are provided in step iii.) in the form of a recipe.

In a particularly preferred embodiment of the present invention, the algorithm for determining the type and quantity of different raw materials in the target food product further provides a score for the determination of the composition of the target food product. Preferably, a score for the determination of the composition of the target food product is provided in step iii.).

Preferably, the score for the determination of the composition of the target food product is a coefficient of determination (R²). In a particularly preferred embodiment of the present invention, the coefficient of determination (R²) is at least 0.8, preferably at least 0.81, preferably at least 0.82, preferably at least 0.83, preferably at least 0.84, preferably at least 0.85, preferably at least 0.86, preferably at least 0.87, preferably at least 0.88, preferably at least 0.89, preferably at least 0.9, preferably at least 0.91, preferably at least 0.92, preferably 0.93, preferably at least 0.94, preferably at least 0.95, preferably at least 0.96, preferably at least 0.97, preferably at least 0.98, preferably at least 0.99.

In a particularly preferred embodiment of the present invention, the score for the determination of the composition of the target food product is based on the deviation, in particular the cumulative deviation, of the nutrient values of the type and quantity of raw materials of the target food product provided in step iii.) from the nutrient values in the target nutrient profile provided in step i.).

Thus, in a preferred embodiment of the present invention, the algorithm compares the determined concentration of each nutrient in the composition of the food product with the target concentration in the target nutrient profile. Preferably, the algorithm provides for each of the nutrient whether the concentration is in the optimal range, the acceptable range or out of the range. In a particular embodiment, the algorithm provides a score for the determined composition of the food product. For example, if the calcium concentration in the food product is in the optimal range, the food product gets 1 point, if the sugar concentration is in the acceptable range, the food product gets 0.5 point, if salt concentration is out of range, the food product gets 0 point. In a preferred embodiment, the sum of all points for each nutrient is the final score of the food product. The score of a food product can be used to express the healthiness of a particular food product. However, according to the present invention, any suitable scoring system expressing the deviation, in particular the cumulative deviation, of the nutrient values of the type and quantity of raw materials of the target food product provided in step iii.) from the nutrient values in the target nutrient profile provided in step i.) is conceivable. As an example only, the score for the determination of the composition of the target food product can range from 0 to 1, 0% to 100%, A to E, I to VI and/or can be represented in the form of a colour code. In the previous non-limiting examples, the upper border, such as 1, 100%, E, VI and/or a particular predetermined colour can represent a perfect match of the nutrient values of the type and quantity of raw materials of the target food product provided in step iii.) with the nutrient values in the target nutrient profile provided in step i.).

In a further preferred embodiment of the present invention, at least two, preferably at least three, preferably at least four, preferably at least five different compositions of the target food product, in particular types and quantities of raw materials of the target food product having a nutrient profile in which the nutrient values of at least 6 nutrients differ from the corresponding nutrient values in the target nutrient profile by at most 30%, are provided in step iii.). According to this embodiment of the present invention, the user is given a variety of suitable compositions for the target food products and advantageously allows to make a decision based on for example but not limited to raw material availability, taste preferences, cultural or religious dietary practices, ethical dietary practices, allergies, medical dietary requirements and/or production technique related issues.

Accordingly, in step a) the composition of a food product is determined and in step b) the thereby obtained information on the type and quantity of raw materials of the target food product is used to prepare a food product having the determined composition.

It is evident for the skilled person that the steps required for the preparation of different types of food products can differ considerably. The steps required for the preparation of a beverage as a target food product based on the type and quantity of raw materials determined by the method according to the present invention significantly differ from the steps required for the preparation of for example flour, pasta or cookies. Depending on the target food product, the preparation in step b) can for example include one or more processing steps selected from drying, hydrating, sterilizing, pasteurizing, cutting, mincing, kneading, grinding, milling, mixing, blending, cooking, baking, freezing and canning. The exact way of preparing the food product having the composition determined in step a) depends on the type of food product and is known to the skilled person.

In a preferred embodiment of the present invention, the food product is a ready-to-eat food product. In another preferred embodiment of the present invention, the food product is an instant food product, such as an instant beverage or a food powder.

In a particularly preferred embodiment of the present invention, the food product is selected from pasta, bakery, confectionary and beverages.

Preferably, the food product is selected from noodles, ravioli, bread, flour, pizza, muesli, granola, chips, cookies, ice-cream, cream, gel, sauce, beverage and smoothie, in particular noodles, bread, flour, pizza, muesli, cookies, ice-cream, cream, gel, sauce, beverage.

A further aspect of the present disclosure is a food product preparable, in particular prepared, by the method for preparing a food product according to the present invention.

In a preferred embodiment, 5 to 80%, preferably 10 to 70%, preferably 30 to 65%, preferably 45 to 60%, of the total energy in the food product is provided by carbohydrates.

In a further preferred embodiment, at most 15%, preferably at most 10%, preferably at most 5%, preferably at most 1%, of the total energy in the food product is provided by free sugars. Preferably, at most 10%, preferably at most 5%, preferably at most 1%, of the total energy in the food product is provided by added isolated sugars.

Preferably, the food product comprises carbohydrates from at least two, preferably at least three, preferably at least four, different raw materials.

In another preferred embodiment, the food product comprises at least one carbohydrate with low glycemic index and/or at least one carbohydrate with high glycemic index. Preferably, the food product comprises at least one slowly digestible carbohydrate and/or at least one quickly digestible carbohydrate. Preferably, the food product comprises at least two, preferably at least three, preferably at least four, different carbohydrates.

In a further preferred embodiment, 5 to 50%, preferably 5 to 40%, preferably 10 to 40%, preferably 5 to 30%, preferably 5 to 20%, preferably 15 to 25%, of the total energy in the food product is provided by proteins.

Preferably, the food product comprises protein from at least two, preferably at least three, preferably at least four, different raw materials.

In a preferred embodiment, the food product has a non-essential amino acids (NAA) to essential amino acids (EAA) ratio of from 10:1 to 1:2, preferably 5:1 to 1:1, preferably 4.5:1 to 3.5:1, preferably 3.5:1 to 2.5:1. In a further preferred embodiment, the food product has a non-essential amino acids (NAA) to essential amino acids (EAA) ratio of at least 2.5, preferably at least 3, preferably at least 3.5, preferably at least 4, preferably at least 4.5, preferably at least 5. Preferably, the food product has a non-essential amino acids (NAA) to essential amino acids (EAA) ratio of at most 10, preferably at most 8, preferably at most 6, preferably at most 5, preferably at most 4.5, preferably at most 4, preferably at most 3.5, preferably at most 3, preferably at most 2.5, preferably at most 2.

In another preferred embodiment, the food product has an alanine or glycine concentration lower than 15 wt.-% based on the total protein content, preferably between 15 wt.-% and 10 wt.-%, preferably between 10 wt.-% and 5 wt.-%, preferably between 5 wt.-% and 2 wt.-%, preferably between 2 wt.-% and 0 wt.-%. Preferably, the food product does not comprise alanine. Preferably, the food product does not comprise glycine.

In a further preferred embodiment, at most 6%, preferably at most 4%, preferably at most 2%, of the total energy in the food product is provided by alanine.

In a preferred embodiment, at most 6%, preferably at most 4%, preferably at most 2%, of the total energy in the food product is provided by glycine.

In a preferred embodiment, the food product comprises at least 30 wt.-% branched chain amino acids (BCAA) based on the total weight of the food product. Preferably, the food product comprises 20 to 30 wt.-%, preferably 10 to 20 wt.-%, preferably 5 to 10 wt.-%, preferably 1 to 5 wt.-%, leucine based on the total weight of the food product.

In a preferred embodiment, 1 to 8%, preferably 1.5 to 5.5%, preferably 2.5 to 4.4%, of the total energy in the food product is provided by branched chain amino acids (BCAA).

In a preferred embodiment, the food product comprises at least 20 wt.-% leucine (Leu) based on the total weight of the food product. Preferably, the food product comprises 15 to 20 wt.-%, preferably 10 to 15 wt.-%, preferably 5 to 10 wt.-%, preferably 1 to 5 wt.-%, leucine based on the total weight of the food product.

In a preferred embodiment, 0.5 to 3%, preferably 0.7 to 2.5%, preferably 1.2 to 2%, of the total energy in the food product is provided by leucine (Leu).

In a preferred embodiment, the food product comprises at least 20 wt.-% glutamine (Gln) based on the total weight of the food product. Preferably, the food product comprises 15 to 20 wt.-%, preferably 10 to 15 wt.-%, preferably 5 to 10 wt.-%, preferably 1 to 5 wt.-%, glutamine based on the total weight of the food product.

In a preferred embodiment, at most 10%, preferably at most 8%, preferably at most 6%, preferably at most 4%, preferably at most 2%, of the total energy in the food product is provided by glutamine (Gln).

In a preferred embodiment, the food product comprises at least 20 wt.-% asparagine (Asn) based on the total weight of the food product. Preferably, the food product comprises 15 to 20 wt.-%, preferably 10 to 15 wt.-%, preferably 5 to 10 wt.-%, preferably 1 to 5 wt.-%, asparagine based on the total weight of the food product.

In a preferred embodiment, at most 10%, preferably at most 8%, preferably at most 6%, preferably at most 4%, preferably at most 2%, of the total energy in the food product is provided by asparagine (Asn).

In a preferred embodiment, the food product comprises at least 40 wt.-% glutamine (Gln) and asparagine (Asn) based on the total weight of the food product. Preferably, the food product comprises 30 to 40 wt.-%, preferably 20 to 30 wt.-%, preferably 10 to 20 wt.-%, preferably 2 to 10 wt.-%, glutamine and asparagine based on the total weight of the food product.

In a preferred embodiment, the food product has a glutamine (Gln) or asparagine (Asn) concentration of lower than 20 wt.-% based on the total protein content, preferably between 20 wt.-% and 15 wt.-%, preferably between 15 wt.-% and 10 wt.-%, preferably between 10 wt.-% and 5 wt.-%, preferably between 5 wt.-% and 0 wt.-%. Preferably, the food product does not comprise glutamine or asparagine.

In a preferred embodiment, at most 16%, preferably at most 10%, preferably at most 8%, preferably at most 6%, preferably at most 4%, of the total energy in the food product is provided by glutamine and asparagine.

In a preferred embodiment, the food product comprises at least 20 wt.-% glutamate (Glu) based on the total weight of the food product. Preferably, the food product comprises 15 to 20 wt.-%, preferably 10 to 15 wt.-%, preferably 5 to 10 wt.-%, preferably 1 to 5 wt.-%, glutamine based on the total weight of the food product.

In a preferred embodiment, 1 to 9%, preferably 2 to 6%, preferably 3 to 5%, of the total energy in the food product is provided by glutamate (Glu).

In a preferred embodiment, the food product comprises at least 20 wt.-% aspartate (Asp) based on the total weight of the food product. Preferably, the food product comprises 15 to 20 wt.-%, preferably 10 to 15 wt.-%, preferably 5 to 10 wt.-%, preferably 1 to 5 wt.-%, aspartate based on the total weight of the food product.

In a preferred embodiment, 1 to 9%, preferably 2 to 6%, preferably 3 to 5%, of the total energy in the food product is provided by aspartate (Asp).

In a preferred embodiment, the food product comprises at least 40 wt.-% glutamate (Glu) and aspartate (Asp) based on the total weight of the food product. Preferably, the food product comprises 30 to 40 wt.-%, preferably 20 to 30 wt.-%, preferably 10 to 20 wt.-%, preferably 2 to 10 wt.-%, glutamate and aspartate based on the total weight of the food product.

In a preferred embodiment, the food product has a glutamate (Glu) or aspartate (Asp) concentration of lower than 20 wt.-% based on the total protein content, preferably between 20 wt.-% and 15 wt.-%, preferably between 15 wt.-% and 10 wt.-%, preferably between 10 wt.-% and 5 wt.-%, preferably between 5 wt.-% and 0 wt.-%. Preferably, the food product does not comprise glutamate or aspartate.

In a preferred embodiment, at most 16%, preferably at most 12%, preferably at most 8%, preferably at most 6%, preferably at most 4%, of the total energy in the food product is provided by glutamate and aspartate.

Preferably, 5 to 50%, preferably 5 to 40%, preferably 10 to 40%, preferably 8 to 30%, preferably 15 to 30%, preferably 20 to 25%, preferably 10 to 20%, preferably 12 to 18%, of the total energy in the food product is provided by fat.

In a particularly preferred embodiment, the food product comprises fat from at least two, preferably at least three, preferably at least four, different raw materials.

In a preferred embodiment, the food product comprises at least one polyunsaturated fatty acid (PUFA). Preferably, the at least one polyunsaturated fatty acid is selected from the group consisting of docosahexanoic acid (DHA), eicosapentaenoic acid (EPA), alpha-linolenic acid (ALA), linoleic acid (LA) and arachidonic acid (ARA). Preferably, the food product comprises at least one omega-3 fatty acid. In a particularly preferred embodiment, the at least one polyunsaturated fatty acid and/or the at least one omega-3 fatty acid is contained in at least one naturally occurring raw material, in particular is derived from algae, sea food, fungus, flaxseeds and/or nuts. Particularly preferred, the at least one polyunsaturated fatty acid and/or the at least one omega-3 fatty acid is derived from at least one oil, in particular from olive oil, flax seed oil, fish oil, almond oil, walnut oil, canola oil, algae oil and/or grape seeds oil. In a further preferred embodiment, the food product comprises at least one polyunsaturated fatty acid and/or the at least one omega-3 fatty acid is an encapsulated form.

In a further preferred embodiment, at most 20%, preferably at most 10%, preferably at most 5%, of the total energy in the food product is provided by saturated fatty acids.

In another preferred embodiment, 0 to 30%, preferably, 3 to 20%, preferably 5 to 15%, preferably 6 to 11% of the total energy in the food product is provided by polyunsaturated fatty acids (PUFAs).

In a preferred embodiment, 0 to 30%, preferably 2 to 20%, preferably 3 to 15%, preferably 4 to 8% of the total energy in the food product is provided by n-6 linoleic acid (18:2).

In a preferred embodiment, 0 to 5%, preferably 0.2 to 2%, preferably 0.3 to 1%, preferably 0.4 to 0.8% of the total energy in the food product is provided by n-3 alpha linolenic acid (18:3).

In a preferred embodiment, 5 to 80%, preferably 10 to 70%, preferably 30 to 65%, preferably 45 to 60%, of the total energy in the food product is provided by carbohydrates and/or 5 to 50%, preferably, 5 to 40%, preferably 5 to 30%, preferably 5 to 20%, preferably 10 to 40%, preferably 15 to 25%, of the total energy in the food product is provided by proteins and/or 5 to 40%, preferably 8 to 30%, preferably 10 to 20%, preferably 12 to 18%, preferably at most 30%, preferably at most 20%, preferably at most 10%, preferably at most 5%, of the total energy in the food product is provided by fat.

In a particularly preferred embodiment, the food product is non-allergenic, in particular does not comprise gluten, lactose, nuts and/or eggs, vegetarian, vegan, kosher and/or halal. Preferably, the food product is non-allergenic, in particular does not comprise gluten, lactose, nuts and/or eggs. In another preferred embodiment, the food product is vegetarian, in particular does not comprise meat. Preferably, the food product is vegan, in particular does not comprise raw material from an animal source. In a further preferred embodiment, the food product is kosher. Preferably, the food product is halal.

In a preferred embodiment, the food product consists of naturally occurring raw materials. Preferably, the food product comprises solely nutrients obtained from a natural source. In a particularly preferred embodiment, the food product does not comprise chemically synthesized nutrients. Preferably, the food product does not comprise added isolated nutrients, in particular does not comprise added isolated vitamins, proteins, carbohydrates, fats, fatty acids, amino acids, nucleotides, antioxidants, trace elements and/or minerals.

In a further preferred embodiment, the food product comprises at least one added isolated nutrient. Preferably, the food product comprises at least two, preferably at least three, preferably at least four added isolated nutrients. Preferably, the food product comprises at most one added isolated nutrient. Preferably, the food product comprises at most two, preferably at most three, preferably at most four added isolated nutrients. Particularly preferred, the added isolated nutrient is selected from the group consisting of vitamins, proteins, carbohydrates, fats, fatty acids, amino acids, nucleotides, antioxidants, trace elements and minerals. In a preferred embodiment of the present invention, the food product comprises added isolated vitamin D and/or vitamin B12. In a further preferred embodiment of the present invention, the food product comprises added isolated NaCl.

In another preferred embodiment, the food product comprises at least one biologically active raw material selected from the group consisting of green tea, matcha tea, turmeric, chlorella, ginger, cardamom, saffron and garlic, in particular consisting of green tea, turmeric, ginger, saffron and garlic.

In a further preferred embodiment, the food product comprises at least one organoleptically and/or visually appealing raw material selected from the group consisting of natural colorant, synthetic colorant, natural aroma, synthetic aroma, vanilla, ginger, chocolate, cacao, lemon, beetroot, orange, spinach, carrot and carob bean.

Preferably, the food product comprises at least one, preferably at least two, preferably at least three, preferably at least four, raw material selected from the group consisting of grains, legumes, seeds, nuts, oils, fruits, vegetables, sea food, meat, meat products, milk and milk products.

In a preferred embodiment, the food product is suitable for human consumption. In a further preferred embodiment, the food product is suitable for animal consumption.

In a preferred embodiment, the food product preparable, in particular prepared, by the method for preparing a food product according to the present invention comprises bran of a grain, for example but not limited to oat bran, rice bran, wheat bran.

In a preferred embodiment, the food product preparable, in particular prepared, by the method for preparing a food product according to the present invention comprises legumes or flours of legumes, for example but not limited to lentils, beans, fava beans, chickpeas, peas or lupins.

In another preferred embodiment, the food product preparable, in particular prepared, by the method for preparing a food product according to the present invention comprises oily seeds, their flours or their pressed cakes or flours of their pressed cakes for example but not limited to flaxseeds, sunflower seeds, pumpkin seeds, sesame seeds, hemp seeds, almond seeds, walnuts seeds, hazelnuts seeds.

In a particularly preferred embodiment, the food product preparable, in particular prepared, by the method for preparing a food product according to the present invention comprises oat bran, rice bran, pea flour and flaxseed.

Preferably, the food product preparable, in particular prepared, by the method for preparing a food product according to the present invention is a dough-based product, in particular a bread, crispy bread, pasta, ravioli, or pizza. Preferably, the dough-based product comprises oat bran, rice bran, pea flour and flaxseed.

Particularly preferred, the food product preparable, in particular prepared, by the method for preparing a food product according to the present invention is a bread comprising wheat germ, flax seeds, rice bran, oats, hemp seeds, salt, and beans.

In another preferred embodiment of the present invention, the food product preparable, in particular prepared, by the method for preparing a food product according to the present invention is granola comprising lentil, sunflower seeds, wheat germ, wheat bran, spinach, yeast, apple, beetroots, carrots, oat bran, curcuma, and cinnamon.

Preferably, the food product preparable, in particular prepared, by the method for preparing a food product according to the present invention is noodles comprising lentil, bamboo fiber, rice bran, oat bran, nutritional yeast, curcuma, and pepper.

In a preferred embodiment, the food product preparable, in particular prepared, by the method for preparing a food product according to the present invention is filled pasta, preferably ravioli, comprising rice bran, sunflower seeds, lentils, spinach, mushroom, onion, oat bran, tomato, broccoli, carrot, yeast, garlic, salt, curcuma, pepper, and matcha.

In a further preferred embodiment, the food product preparable, in particular prepared, by the method for preparing a food product according to the present invention is pizza comprising chicory roots, flax seeds, yeast, rice bran, onion, tomato, red pepper, beans, celery, oat bran, oregano, salt, garlic, oil, and carrot.

A further aspect of the present disclosure pertains to a food product prepared by the method for preparing a food product according to the present invention for use in the prevention and/or treatment of blood glucose related diseases, in particular diabetes, obesity, cardiovascular diseases, Alzheimer's diseases and/or cancer.

In another aspect, the present disclosure relates to the use, in particular non-therapeutical use, of the food product prepared by the method for preparing a food product according to the present invention for gaining weight, for losing weight, for gaining muscle mass, for lowering total cholesterol serum level, for lowering the non-HDL cholesterol serum level, for lowering the total cholesterol/HDL ratio and/or for lowering the triglyceride serum level.

In a preferred embodiment, the present disclosure pertains to the use, in particular non-therapeutical use, of the food product prepared by the method for preparing a food product according to the present invention for gaining weight. In another embodiment, the present disclosure pertains to the use, in particular non-therapeutical use, of the food product prepared by the method for preparing a food product according to the present invention for losing weight. The present disclosure also pertains to the use, in particular non-therapeutical use, of the food product prepared by the method for preparing a food product according to the present invention for gaining muscle mass.

In another embodiment, the present disclosure pertains to the use, in particular non-therapeutical use, of the food product prepared by the method for preparing a food product according to the present invention for lowering total cholesterol serum level, for lowering the non-HDL cholesterol serum level, for lowering the total cholesterol/HDL ratio and/or for lowering the triglyceride serum level.

In the context of the present invention, the terms "raw material" refers to any comestible substance or any additive which can be used for the preparation of a food product. The term encompasses processed or unprocessed naturally occurring raw materials as well as synthetically prepared raw materials. According to the present invention a "raw material" can be a simple substance, such as a specific plant material, for example fruits or vegetables, or a specific animal product, for example meat, milk or egg, but can also be a complex food itself, for example a bread or noodles. In the latter case the "food product" of the present invention can be a complex meal.

In the context of the present invention, the expression "type of raw materials" is used to designate a specific kind of raw material. Typical examples of "types of raw materials" are whole wheat, chickpeas, potatoes, spinach, tomatoes, chicken, salmon.

The term "quantity of raw materials" as used herein refers to a specific amount of raw material. In the context of the present invention, the "quantity of raw materials" can refer to the absolute and relate amount of "raw material" in a composition of a food product.

The term "food" as used herein means any comestible or injectable entity suitable for nutrition which consists of more than one nutrient. According to the present invention, a "food" can be naturally occurring or a product of an industrial process.

In the context of the present invention, the term "food product" is used to designate any prepared comestible or injectable entity consisting of at least two raw materials. A "food product" as used herein can be solid, semi-solid, or liquid. Typical examples of "food products" are bread, noodles, pizza, muesli, cookies, fruit or vegetable juices, instant food and instant beverages.

In the context of the present invention, the term "target food product" means the "food product" which is desired to be obtained. For example, in case a noodle is selected as the "target food product", the algorithm used in the method according to the present invention considers raw materials which are suitable for use in a noodle. The raw materials are not necessarily required to be raw materials commonly used in the production of noodles but can also include unusual raw materials which entirely or partly substitute common raw materials.

The term "meal" as used herein encompasses entities of at least one food product, preferably at least two food products consumed to satisfy the appetite and/or to satisfy the nutritional requirements of an individual. Usually a "meal" is distinguished from a "snack" in terms of the variety of raw materials, foods and/or food products comprises therein and/or in term of quantity. Typical examples for a "meal" are soups, pizzas, pasta dishes, salad bowls, etc.

In the context of the present invention, the term "algorithm" designates a sequence of well-defined unambiguous computer-implementable instructions to perform a specific task. Starting from an initial state and initial "input", the instructions describe a computation that, when executed, proceed through a finite number of well-defined successive steps, eventually producing an "output" and terminating at a final ending state. In a particularly preferred embodiment, the "algorithm" for determining the type and quantity of different raw materials in the target food product manages three modules. In this particular embodiment, the first module is the setting of a target food product and a target nutrient profile. The target nutrient profile contains target concentrations of different nutrient. The target concentration of an individual nutrient in the target nutrient profile does not require to be a single value, but can also be a window of concentration. The window of concentration can further be defined by an optimal concentration range, an acceptable concentration range and out of range. Following example illustrates this particular embodiment for the concentration of the nutrient calcium in a target nutrient profile: optimal concentration range: 100 to 300 mg, acceptable concentration range: 50 to 100 and 300 to 350, out of range: 0 to 50 and higher than 350. Preferably, the second module comprises accessing at least one database, in particular at least one raw material database. The at least one database preferably contains as many different raw materials and corresponding nutrient profiles as possible. The raw materials in the at least one database can be preferentially unprocessed natural raw materials like oat, carrot, etc. They can also be processed raw materials like oat flakes, carrot purees. The at least one database preferably contains the nutrient profile, in particular the concentration of nutrients of each raw material in the database., such as but not limited to calcium, potassium, protein, vitamin A, vitamin C, etc. Particularly preferred, the third module of the algorithm is the limitation of raw material diversity. The algorithm according to the present invention determines the composition of the target food product, like bread, noodles, smoothie, pizza, etc. However, not all raw materials are suitable for each food product. For example, if the target food product is a smoothie preferably fruits, vegetables and/or grains are used in the composition. Raw materials in the at least one database like fish, cheese, etc. are less suitable for smoothie. The algorithm according to the present invention preferably offers the possibility to prefer certain raw materials (in this example fruits and vegetables) or to exclude certain raw materials (in this example fish or cheese). Thus, preferably, this third module of the algorithm is used depending on the target food product for which the composition shall be determined. In other embodiments of the present invention, the third module of the algorithm can also be turned off and the algorithm combines all possible raw materials regardless of their taste compatibility.

The "algorithm" according to the present invention is preferably a software combining above mentioned modules. Preferably, in a first step, the algorithm mixes all possible raw materials in the at least one database in a fixed quantity and determines the nutrient profiles of the raw material compositions. The resulting nutrient profiles of the compositions is then compared to the target nutrient profile. In a second step, the best fitting raw materials are set as constant and their quantities are varied for optimal matching the nutrient concentrations in the target nutrient profile. Thus, in this step, the concentration of the nutrients in the nutrient profile of the composition changes again and the resulting nutrient profile is compared to the target nutrient profile. These two steps (selecting new raw materials and modifying the quantity of each raw material) is preferably performed in an iterative way. In this way, the nutrient profile of the composition step by step approaches to the target nutrient profile. Finally, the determined composition of the target food product is provided by outlining the type and quantity of raw materials which in combination match the target nutrient profile the best, in particular which have a nutrient profile in which the nutrient values of at least 6 nutrients differ from the corresponding nutrient values in the target nutrient profile by at most 30%.

The term "nutrient profile" as used herein refers to the different types and concentrations of nutrients in the target food product. A "target nutrient profile" refers to the a specific predetermined "nutrient profile", namely a specific group of target nutrients in a target concentration. A food product or a raw material having a certain "nutrient profile" or a certain "target nutrient profile" can additionally comprise further nutrients which are not part of the "target nutrient profile". In some embodiments, the "target nutrient profile" can be an optimal nutrient profile for a specific group of individuals or for a specific application. In other embodiments the "target nutrient profile" is a nutrient profile which is improved in comparison to the "nutrient profile" of a common food product, for example improved with regard to the balance of nutrients, the type of nutrients, the amount of nutrients and/or the ratio of nutrients to each other. According to the present invention, the "nutrient profile" of the target food product is characterized in that the nutrient values of at least 6 nutrients differ from the corresponding nutrient values in the "target nutrient profile" by at most 30%.

In the context of the present invention, the term "macronutrient" designates nutrients which are required in large amounts for growth and development of a cell or an organism. Macronutrients may include but are not limited to carbohydrates, fats, proteins, amino acids, salts and water.

The term "micronutrients" as used herein encompasses compounds having a biological function and which are required in only minor or trace amounts. In particular embodiments, the term may include organic and inorganic compounds, such as individual amino acids, nucleotides, fatty acids, vitamins, antioxidants, minerals and trace elements, such as but not limited to copper, iron, zinc, selenium and manganese.

In the context of the present invention, the term "essential amino acids" or in short "EAA" as used herein refers to naturally occurring amino acids which cannot be synthesized by the human body *de novo* at a rate sufficient to meet the nutritional requirements and are therefore required to be taken up by food consumption. "Essential amino acids" for healthy adult humans are histidine, isoleucine, leucine, lysine, methionine, cysteine, phenylalanine, tyrosine, threonine, tryptophan, valine.

The term "semi-essential amino acids" is used in the context of the present invention to designate amino acids which can be synthesized in the human body, wherein the synthesis can be limited under specific physiological or pathophysiological conditions.

In the context of the present invention, the term "non-essential amino acids" or in short "NAA" refers to naturally occurring amino acids which can be synthesized in the human body in sufficient amounts to meet the nutritional requirements. Therefore, "non-essential amino acids" are not necessarily required to be taken up by food consumption. "Non-essential amino acids" for healthy adult humans are alanine, arginine, asparagine, aspartic acid, glutamic acid, glutamine, glycine, proline and serine.

In the context of the present invention, the term "a" is meant to include the meaning of "one" or "one or more".

In the context of the present invention, the term "comprising" preferably has the meaning of "containing" or "including", meaning including the specifically identified elements without excluding the presence of further elements. However, in a preferred embodiment, the term "comprising" is also understood to have the meaning of "consisting essentially of" and in a further preferred embodiment the meaning of "consisting of".

Further preferred embodiments of the invention are subject of the subclaims.

The present invention is further illustrated by way of the following examples and figures.

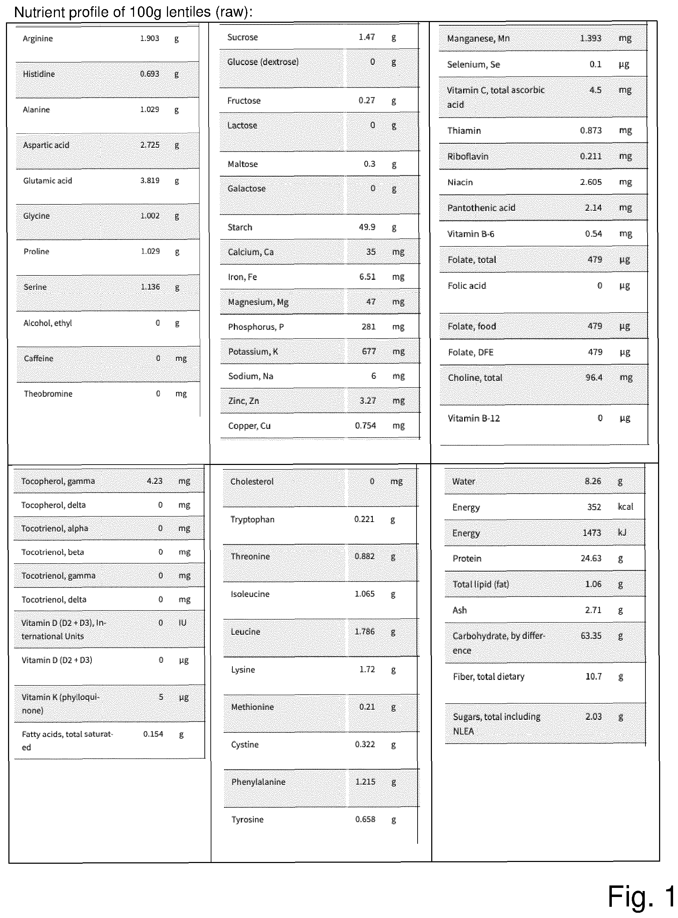

**Figure 1** shows an exemplary nutrient profile of lentils as a raw material obtained from the databank of the U.S. Department of Agriculture on 15 March 2020.

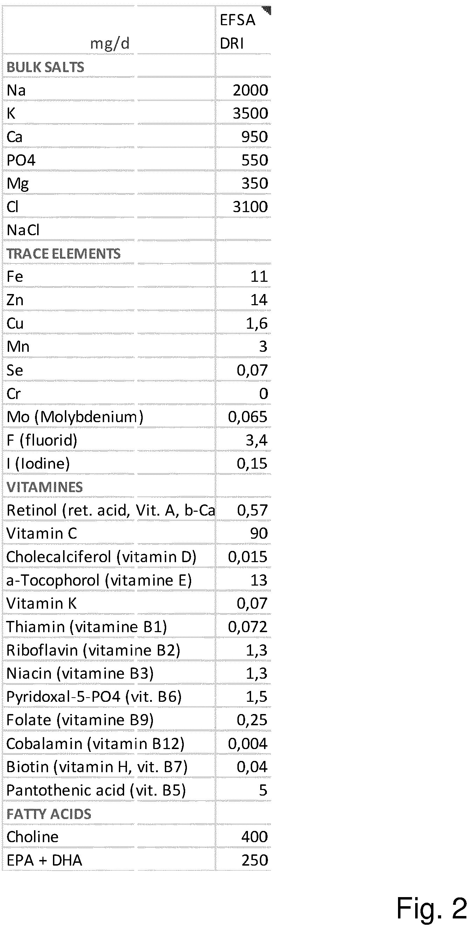

**Figure 2** illustrates an example for a target nutrient profile based on the Dietary Reference Intakes (DRI) from the European Food Safety Authority (EFSA) (Sept 2019).

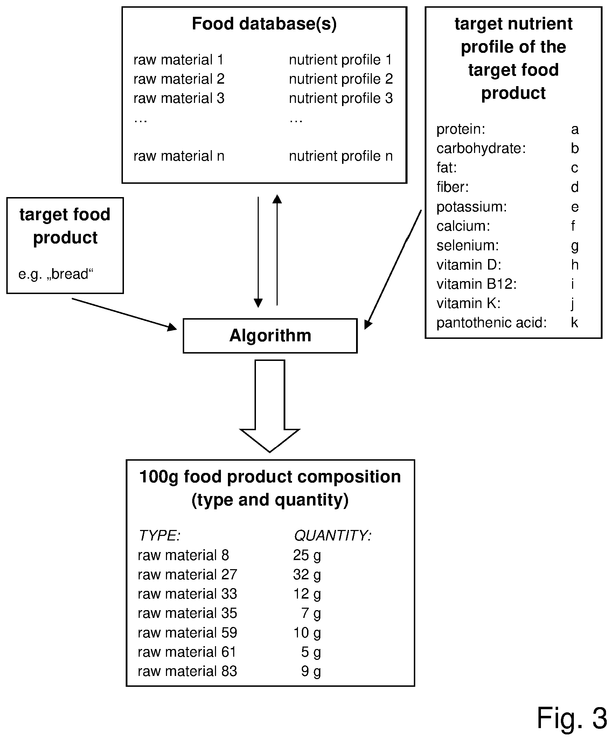

**Figure 3** shows a schematic illustration of the method for determining the composition of a food product.
**Figure 4** shows different compositions of flour determined using the method for determining the composition of a food product.
**Figure 5** illustrates the weight change of participants after 5 weeks consuming food products prepared by the method for preparing a food product according to the invention.
**Figure 6** shows an investigation of the correlation between weight change after 5 weeks consuming food products prepared by the method for preparing a food product according to the invention and age of the participants.
**Figure 7** illustrates an investigation of the correlation between weight change after 5 weeks consuming food products prepared by the method for preparing a food product according to the invention and the starting weight of the participants.
**Figure 8** illustrates the change in blood cholesterol concentration of participants after 5 weeks consuming food products prepared by the method for preparing a food product according to the invention.
**Figure 9** illustrates the change in the feeling of hunger of participants after 5 weeks consuming food products prepared by the method for preparing a food product according to the invention.
**Figure 10** illustrates the change in sleeping quality of participants after 5 weeks consuming food products prepared by the method for preparing a food product according to the invention.

### Example 1: Pizza recipe

Pizza is often considered unhealthy due to a high content of carbohydrates and saturated fats, a low fiber and protein content and a low concentration of polyunsaturated fatty acids. The method for determining the composition of a food product has been used to determine an alternative recipe of a pizza having an improved nutrient profile. Table 1 illustrates the ingredients of a traditional pizza.

**Table 1: Ingredients of a traditional pizza.**

| | |
|---|---|
| Wheat, white [g] | 150 |
| Tomatoes, raw [g] | 50 |
| Cheese, cheddar, sharp, sliced [g] | 50 |
| Oil, olive, salad or cooking [g] | 5 |
| Salt, table [g] | 5 |

Based on the ingredients of traditional pizza the nutrient profile is determined. Subsequently, the method for determining the composition of a food product is used to determine an alternative composition of pizza having an improved nutrient profile in comparison to traditional pizza. The composition of a pizza determined by the method for determining the composition of a food product in comparison to the composition of traditional pizza is given in table 2.

**Table 2: Comparison of ingredients of a traditional pizza and of a pizza determined by the method for determining the composition of a food product.**

| | Traditional pizza | Pizza Recipe 1: | Pizza Recipe 2: |
|---|---|---|---|
| Wheat, white [g] | 150 | | |
| Wheat, whole [g] | | 75 | 28 |
| Oat [g] | | 75 | 45 |
| Chickpeas [g] | | | 45 |
| Tomatoes, raw [g] | 50 | 50 | 20 |
| Tomatoes, sundried [g] | | | 30 |
| Cheese, cheddar, sharp, sliced [g] | 50 | | |
| Cheese, low fat, cheddar or Colby [g] | | 50 | 45 |
| Oil, olive, salad or cooking [g] | 5 | 1 | |
| Oil, flaxseed, cold pressed [g] | | 4 | |
| Salt, table [g] | 5 | 5 | 5 |
| Seeds, hemp seeds [g] | | | 3 |
| Mushrooms, white [g] | | | 45 |
| Broccoli [g] | | | 24 |
| Leek [g] | | | 10 |
| Spinach [g] | | | 30 |
| Wheat, germ oil [g] | | | 1 |
| Yeast [g\|] | | | 8 |

The nutrient profile of the pizza obtained by the method for determining the composition of a food product is improved in comparison to the traditional pizza by about 29% (Recipe 1). Recipe 2 provides an alternative pizza determined by the method for determining the composition of a food product having an even more improved nutrient profile in comparison to traditional pizza (62% improvement). Table 3 illustrates exemplary nutritional parameters of three recipes.

**Table 3: Nutritional parameters of traditional pizza and pizza determined by the method for determining the composition of a food product.**

| Parameter | Traditional pizza | Pizza Recipe 1: | Pizza Recipe 2: |
|---|---|---|---|
| Fibre [g] | 5 | 17 | 21 |
| Carbohydrate [% of total energy] | 58 | 62 | 62 |
| Protein [% of total energy] | 14 | 21 | 25 |
| Fat [% of total energy] | 26 | 21 | 25 |
| Saturated fat [% of total energy] | 12 | 5 | 5 |
| PUFA [% of total energy] | 2 | 7 | 7 |
| Energy density [calory/100 g product] | 382 | 326 | 202 |

The recipes for pizza obtained by the method for determining the composition of a food product have lower saturated fats, higher fiber content, higher protein content, higher PUFA content and lower calories. In particular the pizza obtained by recipe 2 has a significantly improved nutritional profile in comparison to traditional pizza and is, advantageously, furthermore a low caloric product.

### Example 2: Improvement of the nutrient profile of a target food product

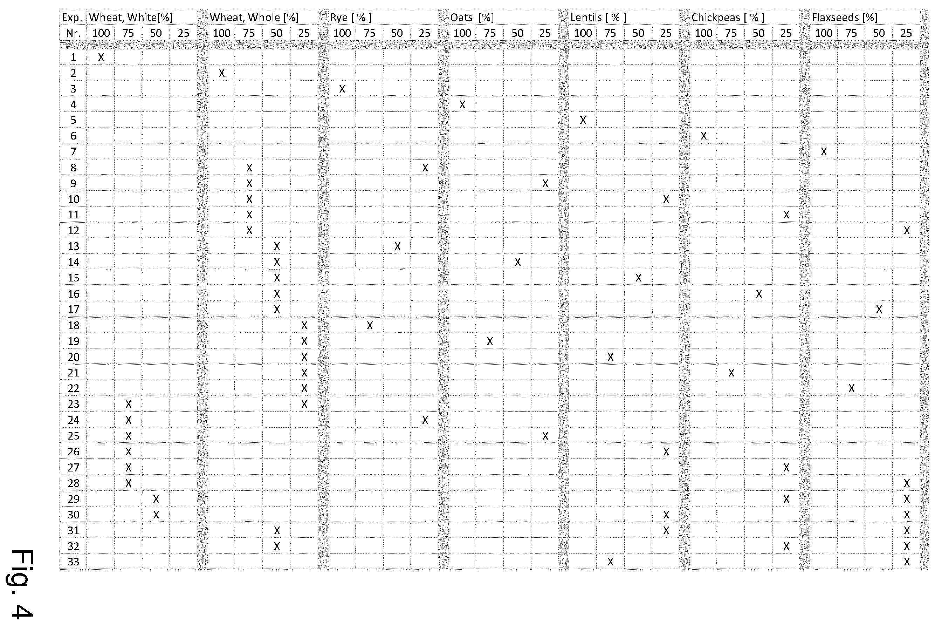

In order to demonstrate how the method for determining the composition of a food product can improve the nutrient profile of well-known traditional food products, different combinations of raw materials for flour as a target food product were determined using the method for determining the composition of a food product (see Figure 4). The improvement of the nutrient profile in comparison to white wheat flour as a control is given in Table 4.

**Table 4: Improvement of the nutrient profile of flour in comparison to white wheat flour as a control**

| *Control* | *Exp. 2* | *Exp. 3* | *Exp. 4* | *Exp. 5* | *Exp. 6* | *Exp. 7* | *Exp. 8* | *Exp. 9* | *Exp. 10* |
|---|---|---|---|---|---|---|---|---|---|
| - | +43.5% | +21.7% | +69.5% | +60.8% | +65.2% | +86.9% | +43.4% | +43.4% | +65.2% |

| *Exp. 11* | *Exp. 12* | *Exp. 13* | *Exp. 14* | *Exp. 15* | *Exp. 16* | *Exp. 17* | *Exp. 18* | *Exp. 19* | *Exp. 20* |
|---|---|---|---|---|---|---|---|---|---|
| +52.1% | +60.8% | +34.7% | +65.2% | +69.5% | +69.5% | +78.2% | +26.0% | +69.5% | +69.5% |

| *Exp. 21* | *Exp. 22* | *Exp. 23* | *Exp. 24* | *Exp. 25* | *Exp. 26* | *Exp. 27* | *Exp. 28* | *Exp. 29* | *Exp. 30* |
|---|---|---|---|---|---|---|---|---|---|
| +65.2% | +86.9% | +8.6% | +4.3% | +13.0% | +30.4% | +13.0% | +43.4% | +69.5% | +73.9% |

| *Exp. 31* | *Exp. 32* | *Exp. 33* | | | | | | | |
|---|---|---|---|---|---|---|---|---|---|
| +86.9% | +86.9% | +91.3% | | | | | | | |

**Table 5: Exemplary compositions of flour and improvement of the nutrient profile in comparison to white wheat flour as a control**

| *Raw material* | *Control* | *Exp.2* | *Exp. 4* | *Exp. 19* | *Exp. 29* | *Exp. 30* | *Exp.31* |
|---|---|---|---|---|---|---|---|
| Wheat, white | 100% | | | | 50% | 50% | |
| Wheat, whole | | 100 % | | 25% | | | 50% |
| Lentils | | | | | | 25% | 25% |
| Flaxseeds | | | | | 25% | 25% | 25% |
| Oats | | | 100% | 75% | | | |
| Chickpeas | | | | | 25% | | |
| Improvement to nutrient profile of control | - | +43.5% | +69.5% | +69.5% | +69.5% | +73.9% | +86.9% |

As apparent from Figure 4 and Table 5, the algorithm of the method for determining the composition of a food product can determine the composition of nutritionally improved flour as a target food product based on a target nutrient profile and a group of nutrient profiles of raw materials in the at least one database.

In cases in which more than one composition equally differs from the target nutrient profile all possibilities will be provided to the user (see Exp. 4, Exp. 19 and Exp. 29).

### Example 3: Clinical trials

A clinical study has been performed with a food product obtained by the method according to the present invention. In this study, the glucose to insulin ratio (G:I ratio) was used to measure the effect of the food product on human metabolism. Since the food product obtained by the method according to the present invention reduces the serum insulin level, measurement of the G:I ratio may be used as a simple test for evaluating the therapeutic effectiveness of the food product.

### Example 4: Effect of food products on body weight

The aim of the experiment was to investigate the influence of food products prepared by the method according to the present invention on body weight. The participants we advised to integrate multiple food products prepared by the method according to the present invention into their everyday diet. The participants consumed the food products for an investigation period of 5 weeks. Multiple products prepared by the method according to the present invention were offered to the participants so as to provide a decent variety in the everyday diets and to avoid that the participants refrain from consuming the products prepared by the method according to the invention after a certain while.

Following food products were designed and produced according to the invention:
Bread: Wheat germ, flax seeds, rice bran, oats, hemp seeds, salt, beans.
Granola: Lentil, sunflower seeds, wheat germ, wheat bran, spinach, yeast, apple, beetroots, carrots, oat bran, curcuma, cinnamon.
Noodle: Lentil, bamboo fiber, rice bran, oat bran, yeast, curcuma, pepper.
Ravioli: rice bran, sunflower seeds, lentils, spinach, mushroom, onion, oat bran, tomato, broccoli, carrot, yeast, garlic, salt, curcuma, pepper, matcha.
Pizza: chicory roots, flax seeds, yeast, rice bran, onion, tomato, red pepper, beans, celery, oat bran, oregano, salt, garlic, oil, carrot.

All food products in the study were developed using the method according to the invention. In each food product the concentrations and the ratios of about 60 single nutrients were optimised. Thus, food products were nutritionally balanced with respect of about 60 nutrients. For that, the nutrient profile of each raw material has been considered. The special algorithm was used to determine the types of raw material used and in which quantities the raw materials are to be used in order to obtain optimally balanced food product compositions. In this way, nutritionally perfectly balanced food products were obtained.

66 males and females in the age of 19 to 72 were randomly selected as participants of the study. Some of the participants had normal Body Mass Index (BMI), some participants had overweight, some participants had health complaints (illnesses), while others did not have any health complaints. In total, the group of study participants represented a random reflection of the population.

The study participants were divided in two groups:
**Group A** consisted of 33 participants which were advised to integrate the above food products into their daily diet. No other obligations were made, e.g. no calorie restriction, no obligation for doing more sports whatsoever. The participants of group A were told, that if they like to eat bread, they shall eat bread determined and prepared according to the present invention instead of buying usual bread in supermarket. If they like to eat ravioli, they shall eat ravioli determined and prepared according to the present invention instead of buying usual ravioli in supermarket, etc. There was also no restriction to eat anything else than what they normally eat.

**Group B** consisted of 33 participants as well. The participants of group B were advised to integrate food products determined and prepared according to the present invention into their diet in the same way as described for group A. In addition, the participants of group B were told to practice following 5 routines: 1) to drink water before each meal, 2) to eat food slowly, minimum in 20 minutes, 3) not to eat any food after 19 p.m. in the evening, 4) to eat maximum 3 times a day and not in between, and 5) to eat salad in addition to the food products prepared by the method according to the present invention. The participants had no other obligations, like no calorie restriction, no obligation for doing sports, etc.

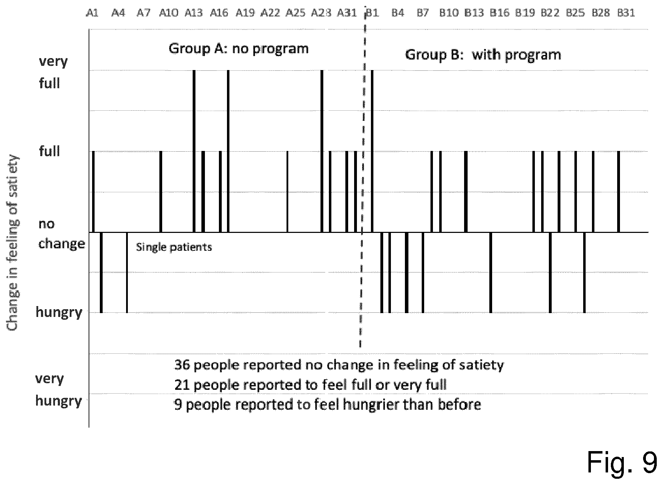

Participants consuming the food products prepared by the method according to the present invention reported that the food products are giving high satiating effect without getting hungry again quickly (see Figure 9). The food products supplied the body of the participants with necessary single nutrients. The participants could thus lose weight without feeling hungry and without lacking essential nutrients for their body.

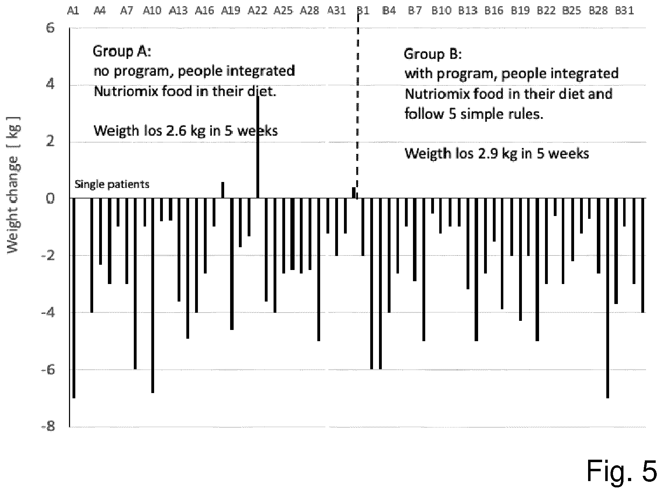

The results demonstrate that 63 of 66 people lost weight within the investigation period of 5 weeks (Figure 5). Two people gained weight, both below 0.5 kg during the 5 weeks period. One woman had her menstruation during the measurement, so that her body accumulated water at the time point of measurement, therefore it appears as if she gained 3.6 kg. Two days later her weight was 2 kg less again. That particular person was excluded from the assessment of the food product induced change of body weight.

Participants in **group B** lost more weight (in average -2.9 kg) than in **group A** (in average -2.6 kg). This also demonstrates that simple behavioural changes contributed to and intensified the positive effect of the food products prepared by the method according to the present invention. However, the food products prepared by the method according to the invention alone are sufficient to generate positive effect in the body **(group A).**

The results of the study were further analysed whether the effect of the food products depends on the age of participants.

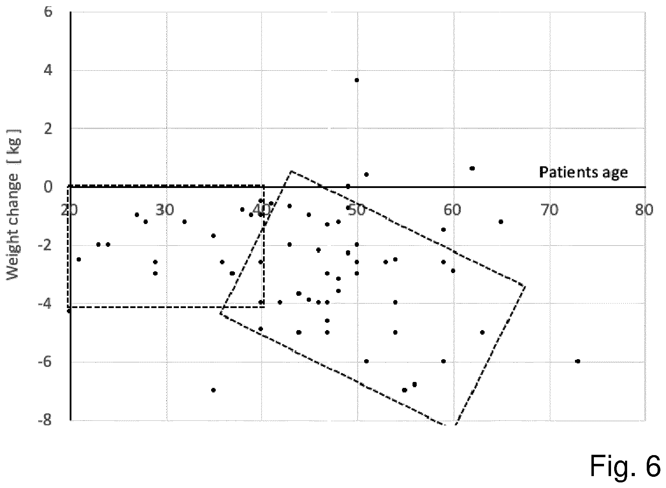

Data analysis demonstrates that nearly all participants lost weight (Figure 5). In addition, participants in the age of 19 to 40 seem to lose less weight (in average about -2 kg) than the participants in the age of 40 to 60 (in average about -4 kg). An explanation for this difference could be that the participants did not have any particular obligation to follow. They were free to eat whatever they like to eat. Younger participants might tend to eat more junk food, more industrially over-processed food. This difference in eating habits between younger and older participants is considered the main reason for the observed difference in weight loss (Figure 6). Nevertheless, it can be concluded that the food products according to the invention caused similar effects in younger and older participants.

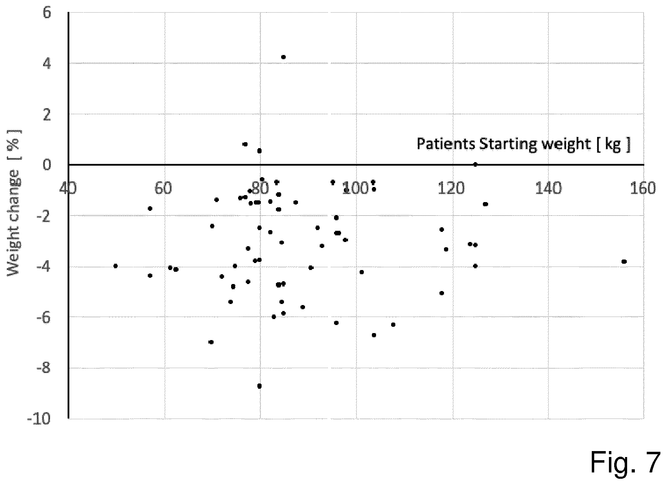

In a next step, it was also assessed whether the observed weight loss depends on the starting body weight of the participants; in other words, whether weight loss depends on the Body Mass Index (BMI) of the participants. Data analysis shows that weight loss does not correlate with starting body weight. Skinny and overweight participants both lost weight equally well by consuming food products according to the invention (Figure 7).

### Example 5: Effect of food products on blood cholesterol level

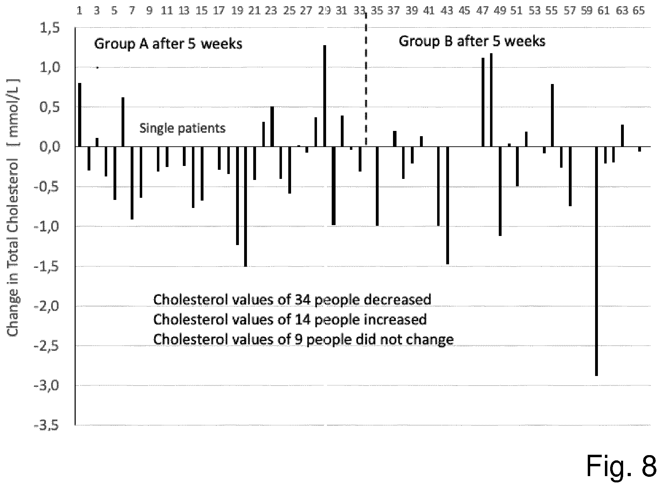

The changes in blood cholesterol level of the participants were followed as well. Blood samples were taken from the participants before they have started consuming food products prepared by the method according to the invention. The frame of the study was the same as described in Example 4. Another blood sample was taken from the participants after 5 weeks consuming food products prepared by the method according to the present invention. Although changes in blood parameters usually take longer, still a tendence for a decrease in cholesterol levels could be observed. Surprisingly, the decrease could be seen already in such a short time frame (Figure 8).

### Example 6: Effect of food products on life quality

The study further included monitoring of additional parameters which could be influence by the consumption of food products prepared by the method according to the present invention. In this regard, the participants were asked a number of questions on a weekly basis and the changes in their subjective feelings were recorded. Particularly, the participants were asked whether they feel more or less hungry than usual during the day by consuming food products prepared by the method according to the invention.

Most of the participants reported that the food products prepared by the method according to the invention have a high satiating effect. They were not feeling in such a way hungry like before. Most importantly, they were not getting hunger crisis as partially observed before the study. After consuming the food products, the peaks of hunger crisis were reduced or gone (Figure 9). Hunger crisis or the feeling of hunger is a result of many parameters like what else the participants otherwise eat, such as sweets, simple carbohydrates etc. It has to be considered that during the test, there were no other dietary obligations for the participants. They could eat sweets as they wished. The feeling of hunger is mainly influence depending on the frequency and quantity of sweets or simple carbohydrates consumed by the participants. Still, there is a trend towards feeling less hungry upon consumption of food products prepared by the method according to the invention. This might result from the balanced nutritional profile of the food products. In the food products provided in the study, there was no single nutrient limiting. Thus, with each meal the test persons were consuming a complete set of balanced nutrients. Consequently, the body is supplied with all necessary nutrients and there is no intuitive desire anymore to compensate a missing nutrient. That might be the reason why the feeling of hunger was reduced or gone.

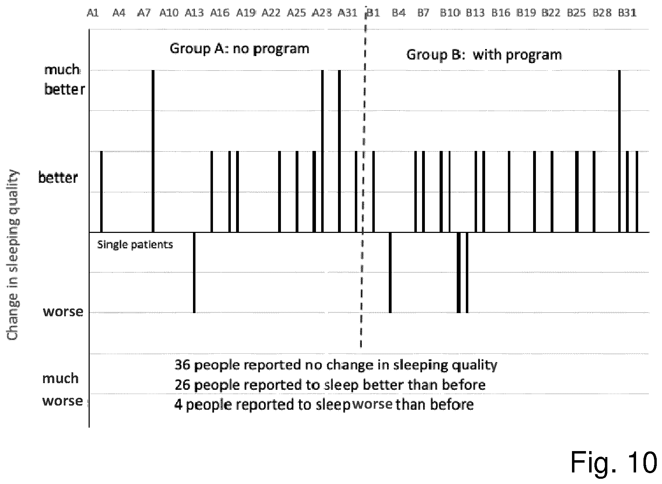

A further question asked to the participants was whether they have recognised a change in their sleeping quality. The majority of the participants indicated that there was no recognizable difference in sleeping quality. Sleeping is another complex process. Many factors influence the sleeping quality. Still, a trend could be observed towards an improvement of the sleeping quality of participants (Figure 10). This positive outcome might be due to the consumption of balanced food and thus not overeating and sleeping better.

## Claims

1. A computer-implemented method for preparing a food product, comprising the steps:
a) determining the composition of a food product, comprising the steps:
i.) providing at least one database comprising nutrient profiles of raw materials from a natural source, a target food product and a target nutrient profile of the target food product,
wherein the target nutrient profile is a specific group of at least 6 target nutrients each in a target concentration, wherein the target nutrient profile has a more balanced ratio of nutrients and a higher diversity of nutrients than a common food product corresponding to the target food product,
ii.) applying an algorithm for determining the type and quantity of at least four different raw materials selected from the group consisting of grains, legumes, seeds, nuts, oils, fruits, vegetables, sea food, meat, meat products, milk and milk products in the target food product on a group of nutrient profiles of raw materials in the at least one database,
wherein the algorithm has been trained using a machine learning procedure,
iii.) providing the determined type and quantity of the at least four different raw materials of the target food product having a nutrient profile in which the nutrient concentrations of at least 6 nutrients differ from the corresponding nutrient concentrations in the target nutrient profile by at most 30%,
wherein the types and quantities of the at least four different raw materials for preparing the target food product are provided in the form of a recipe,
b) preparing a food product having the composition determined in step a) from the at least four different raw materials.

2. The method according to claim 1, wherein the group of nutrient profiles of raw materials encompasses nutrient profiles of selected raw materials in the at least one database, preferably of raw materials selected from non-allergic raw materials, meat-free raw materials, raw materials free of animal products, kosher raw materials and/or halal raw materials.

3. The method according to claim 1 or 2, wherein the target nutrient profile of the food product is a nutrient profile for a specific group of individuals or for a specific application.

4. The method according to claim 3, wherein the target nutrient profile of the food product is a gender-specific nutrient profile.

5. The method according to any one of claims 1 to 3, wherein the food product is selected from noodles, bread, flour, pizza, muesli, granola, chips, cookies, ice-cream, cream, gel, sauce, beverage.

## Patentansprüche

1. Computer-implementiertes Verfahren zur Herstellung eines Nahrungsmittels, umfassend die Schritte:
a) Bestimmen der Zusammensetzung eines Nahrungsmittels, umfassend die Schritte:
i.) Bereitstellen mindestens einer Datenbank, umfassend Nährstoffprofile von Rohmaterialien aus einer natürlichen Quelle, ein Ziel-Nahrungsmittel und ein Ziel-Nährstoffprofil des Ziel-Nahrungsmittels,
wobei das Ziel-Nährstoffprofil eine spezifische Gruppe von mindestens 6 Ziel-Nährstoffen jeweils in einer Ziel-Konzentration ist, wobei das Ziel-Nährstoffprofil ein ausgewogeneres Verhältnis von Nährstoffen und eine höhere Vielfalt von Nährstoffen aufweist als ein gewöhnliches Nahrungsmittel, das dem Ziel- Nahrungsmittel entspricht,
ii.) Anwenden eines Algorithmus zur Bestimmung der Art und Menge von mindestens vier verschiedenen Rohmaterialien, ausgewählt aus der Gruppe bestehend aus Getreide, Hülsenfrüchten, Samen, Nüssen, Ölen, Früchten, Gemüse, Meeresfrüchten, Fleisch, Fleischprodukten, Milch und Milchprodukten in dem Ziel-Nahrungsmittel auf eine Gruppe von Nährstoffprofilen von Rohmaterialien in der mindestens einen Datenbank,
wobei der Algorithmus unter Verwendung eines maschinellen Lernverfahrens trainiert worden ist,
iii.) Bereitstellen der ermittelten Art und Menge der mindestens vier verschiedenen Rohmaterialien des Ziel-Nahrungsmittels, die ein Nährstoffprofil aufweisen, bei dem die Nährstoffkonzentrationen von mindestens 6 Nährstoffen um höchstens 30% von den entsprechenden Nährstoffkonzentrationen im Ziel-Nährstoffprofil abweichen,
wobei die Arten und Mengen der mindestens vier verschiedenen Rohmaterialien zur Herstellung des Ziel-Nahrungsmittels in Form eines Rezeptes bereitgestellt werden,
b) Herstellen eines Nahrungsmittels, aufweisend die in Schritt a) ermittelte Zusammensetzung aus den mindestens vier verschiedenen Rohmaterialien.

2. Verfahren gemäß Anspruch 1, wobei die Gruppe der Nährstoffprofile von Rohmaterialien Nährstoffprofile ausgewählter Rohmaterialien in der mindestens einen Datenbank beinhaltet, bevorzugt von Rohmaterialien ausgewählt aus nicht-allergenen Rohmaterialien, fleischfreien Rohmaterialien, Rohmaterialien frei von tierischen Produkten, koscheren Rohmaterialien und/oder Halal-Rohmaterialien.

3. Verfahren gemäß Anspruch 1 oder 2, wobei das Ziel-Nährstoffprofil des Nahrungsmittels ein Nährstoffprofil für eine bestimmte Personengruppe oder für eine bestimmte Anwendung ist.

4. Verfahren gemäß Anspruch 3, wobei das Ziel-Nährstoffprofil des Nahrungsmittels ein geschlechtsspezifisches Nährstoffprofil ist.

5. Verfahren gemäß einem der Ansprüche 1 bis 3, wobei das Nahrungsmittel ausgewählt ist aus Nudeln, Brot, Mehl, Pizza, Müsli, Granola, Chips, Keksen, Speiseeis, Sahne, Gel, Soße, Getränk.

## Revendications

1. Une méthode mise en œuvre par ordinateur pour préparer un produit alimentaire, comprenant les étapes :
a) déterminer la composition d'un produit alimentaire, comprenant les étapes :
i.) fournir au moins une base de données comprenant les profils nutritionnels des matières premières de source naturelle, un produit alimentaire cible et un profil nutritionnel cible du produit alimentaire cible,
dans lequel le profil nutritionnel cible est un groupe spécifique d'au moins 6 nutriments cibles chacun dans une concentration cible, dans lequel le profil nutritionnel cible a un rapport plus équilibré de nutriments et une plus grande diversité de nutriments qu'un produit alimentaire courant correspondant au produit alimentaire cible,
ii.) appliquer un algorithme pour déterminer le type et la quantité d'au moins quatre matières premières différentes choisies parmi le groupe constitué de céréales, légumineuses, graines, noix, huiles, fruits, légumes, fruits de mer, viande, produits à base de viande, lait et produits laitiers dans le produit alimentaire cible sur un groupe de profils nutritionnels de matières premières dans la au moins base de données,
dans lequel l'algorithme a été entraîné en utilisant une procédure d'apprentissage machine,
iii.) fournir le type et la quantité déterminés d'au moins quatre matières premières différentes du produit alimentaire cible ayant un profil nutritionnel dans lequel les concentrations nutritionnelles d'au moins 6 nutriments diffèrent des concentrations nutritionnelles correspondantes dans le profil nutritionnel cible de 30 % au moins,
dans lequel les types et les quantités d'au moins quatre matières premières différentes pour la production du produit alimentaire cible sont fournis sous la forme d'une recette,
b) préparer un produit alimentaire ayant la composition déterminée à l'étape a) à partir des au moins quatre matières premières différentes.

2. La méthode selon la revendication 1, dans lequel le groupe de profils nutritionnels des matières premières inclut les profils nutritionnels des matières premières choisies dans la au moins base de données, préférablement des matières premières choisies parmi les matières premières non allergènes, les matières premières sans viande, les matières premières sans produits animaux, les matières premières casher et/ou les matières premières halal.

3. La méthode selon la revendication 1 ou 2, dans laquelle le profil nutritionnel cible du produit alimentaire est un profil nutritionnel pour un groupe spécifique d'individus ou pour une application spécifique.

4. La méthode selon la revendication 3, dans lequel le profil nutritionnel cible du produit alimentaire est un profil nutritionnel spécifique au sexe.

5. La méthode selon l'une quelconque des revendications 1 à 3, dans lequel le produit alimentaire est choisi parmi les nouilles, le pain, la farine, la pizza, le muesli, le granola, les chips, les biscuits, la crème glacée, la crème, le gel, la sauce, la boisson.
